# EUROPEAN PATENT APPLICATION

(11) **EP 4 241 738 A1**
(43) Date of publication of application: **13.09.2023**
(21) Application number: 23160240.0
(22) Date of filing: 06.03.2023
(51) Int. Cl.: A61F 2/30, A61F 2/34, A61L 27/56, B33Y 80/00

(54) **NON-POLYGONAL POROUS STRUCTURE**

(30) Priority: 07.03.2022 US 202263268958 P
(71) Applicant: Waldemar Link GmbH & Co. KG, 22339 Hamburg (DE)
(72) Inventor: Link, Helmut D., 22339 Hamburg (DE)
(74) Representative: Alatis

(57) **Abstract**

The disclosure includes a porous implantable structure, that includes substantially regularly arranged elementary cells, wherein the elementary cells include interior spaces that form a plurality of interconnected pores, the elementary cells include basic elements arranged in layers, wherein the basic elements are configured to form a non-polygonal shape of each of the plurality of interconnected pores, wherein each layer is offset with respect to an adjacent layer.

## Description

### CROSS-REFERENCE TO RELATED APPLICATIONS

This application claims priority to U.S. Provisional Application No. 63/268,958, filed March 7, 2022, the contents of which are incorporated by reference in the present disclosure in their entirety.

### BACKGROUND

The present disclosure relates to implantable bone implants, specifically a non-polygonal porous structured element that can be a portion of, or form the entirety of, a prosthesis and/or an implant for joint replacement, bone repair, restoration of function, etc.

Medical implants are devices that may be placed inside the body. A prosthesis is an artificial device that can replace or augment a missing or impaired part of the body. Previously, prosthetic manufacturing has been labor intensive, manual and costly. However, recently, 3D additive manufacturing methods have evolved and revolutionized prosthetic manufacturing.

Improved prosthetic elements and bone implants made using additive manufacturing are known for having a lattice like porous structure. Generally, the porous structure possesses a geometrical shape that confers resistance to force and promotes biologic fixation to surrounding bony tissue. The porous material is configured such as to encourage ingrowth of surrounding bone into the pore spaces of the porous material. Typically, the porous material and any coating applied thereto may comprise titanium alloys, pure titanium, cobalt chromium, stainless steel, tantalum, zirconium and other biocompatible materials.

Porous structures can be formed by additive manufacturing methods like Selective Laser Melting (SLM) or Electron Beam Melting (EBM). These methods allow for making porous metal products by building layers and solidifying material from powder to solid by means of a melting process. Using additive manufacturing offers relative precision and a range of desired structures may be achieved. For example, acetabular cups containing porous structures made by additive manufacturing can be formed. In such acetabular cups, a porous structure made of cells in an arrangement can be provided. In this example, cells of the porous structure are configured in a polygon configuration, which provides a shape in various planes.

Elementary cells in different layers, wherein the elementary cells are formed by interconnected basic elements, can be arranged. Also, each of the layers can be shifted such that the basic elements of one layer are rotated in respect to its adjacent lower layer. This shifted arrangement of the layers, which were otherwise regularly arranged basic elements, provides an improvement in biocompatibility.

However, non-polygonal porous structures as portions of, components of, or entire structures of, bone implants have not been disclosed. These non-polygonal structures can provide more advantageous biological fixation as compared to polygonal structures due at least in part to the non-polygonal structures are more similar to the structure of real, natural bone, including for example trabecular bone material. Thus, what is desired are bone implants with non-polygonal porous, structural elements, that address known deficiencies.

### BRIEF SUMMARY OF THE DISCLOSURE

In accordance with one or more embodiments, devices and methods are provided.

The disclosure includes a porous implantable structure, that includes substantially regularly arranged elementary cells, wherein the elementary cells include interior spaces that form a plurality of interconnected pores, the elementary cells include basic elements arranged in layers, wherein the basic elements are configured to form a non-polygonal shape of each of the plurality of interconnected pores, wherein each layer is offset with respect to an adjacent layer.

These and other advantages of the disclosure will be apparent to those of ordinary skill in the art by reference to the following detailed description and the accompanying drawings.

### BRIEF DESCRIPTION OF THE DRAWINGS

The accompanying drawings, which are incorporated in and constitute a part of this specification, illustrate embodiments of the disclosure, and, together with the summary given above, and the detailed description of the embodiments below, serve as a further explanation and disclosure to explain and/or illustrate embodiments of the disclosure.
FIG. 1 is a magnified, cross-sectional view of an embodiment of a non-polygonal structure of the disclosure.
FIGs. 2A-2C are views of repeated elementary cells of an embodiment of a porous structure of a bone implant with non-polygon shaped pores that are substantially circular.

### DETAILED DESCRIPTION

To facilitate the understanding of this disclosure a number of terms of in quotation marks are defined below. It is noted that the drawings of the present application are provided for illustrative purposes only and, as such, the drawings are not drawn to scale. It is also noted that like and corresponding elements are referred to by like reference numerals.

In the following description, numerous specific details are set forth, such as particular structures, components, materials, dimensions, processing steps and techniques, in order to provide an understanding of the various embodiments of the present application. However, it will be appreciated by one of ordinary skill in the art that various embodiments of the present application may be practiced without these specific details. In other instances, well-known structures or processing steps have not been described in detail in order to avoid obscuring the present application.

Throughout this disclosure, unless the context requires otherwise, the word "comprise" and variations such as "comprises" and "comprising" will be understood to imply the inclusion of a stated integer or step or group of integers or steps but not the exclusion of any other integer or step or group of integers or steps.

It will be understood that when an element as a layer, region or substrate is referred to as being "on" or "over" another element, it can be directly on the other element or intervening elements may also be present. In contrast, when an element is referred to as being "directly on" or "directly over" another element, there are no intervening elements present. It will also be understood that when element is referred to as being "beneath" or "under" another element, it can be directly beneath or under the other element, or intervening elements ay be present. In contrast, when an element is referred to as being "directly beneath" or "directly under" another element, there are no intervening elements present.

As used herein, the term "substantially" or "substantial", is equally applicable when used in a negative connotation to refer to the complete or near complete lack of an action, characteristic, property, state, structure, item, or result. For example, a surface that is "substantially" flat would either be completely at, or so nearly flat that the effect would be the same as if it were completely flat.

As used herein, terms defined in the singular are intended to include those terms defined in the plural and vice versa.

As used in this specification and its appended claims, terms such as "a", "an" and "the" are not intended to refer to only a singular entity, but include the general class of which a specific example may be used for illustration, unless the context dictates otherwise. The terminology herein is used to describe specific embodiments of the invention, but their usage does not delimit the invention, except as outlined in the claims.

Unless otherwise indicated, all numbers expressing quantities of ingredients, properties such as molecular weights, reaction conditions, and so forth as used in the specification and claims are to be understood as being modified in all instances by the term "about." Accordingly, unless indicated to the contrary, the numerical parameters in the specification and claims are approximations that may vary depending upon the desired properties sought to be obtained by the present invention. At the very least, and without limiting the application of the doctrine of equivalents to the scope of the claims, each numerical parameter should at least be construed in light of the number of reported significant digits and by applying ordinary rounding techniques. Notwithstanding that the numerical ranges and parameters describing the broad scope of the invention are approximations, the numerical values in the specific examples are reported as precisely as possible. Any numerical value, however, inherently contains standard deviations that necessarily result from the errors found in the numerical value's testing measurements.

Thus, reference herein to any numerical range expressly includes each numerical value (including fractional numbers and whole numbers) encompassed by that range. To illustrate, reference herein to a range of "at least 50" or "at least about 50" includes whole numbers of 50, 51, 52, 53, 54, 55, 56, 57, 58, 59, 60, etc., and fractional numbers 50.1, 50.2 50.3, 50.4, 50.5, 50.6, 50.7, 50.8, 50.9, etc. In a further illustration, reference herein to a range of "less than 50" or "less than about 50" includes whole numbers 49, 48, 47, 46, 45, 44, 43, 42, 41, 40, etc., and fractional numbers 49.9, 49.8, 49.7, 49.6, 49.5, 49.4, 49.3, 49.2, 49.1, 49.0, etc. In yet another illustration, reference herein to a range of from "5 to 10" includes whole numbers of 5, 6, 7, 8, 9, and 10, and fractional numbers 5.1, 5.2, 5.3, 5,4, 5,5, 5.6, 5.7, 5.8, 5.9, etc.

In the discussion and claims herein, the tern "about" indicates that the value listed may be somewhat altered, as long as the alteration does not result in nonconformance of the process or structure to the illustrated embodiment. For example, for some elements the term "about" can refer to a variation of ±0.1%, for other elements, the term "about" can refer to a variation of ±1% or ±10%, or any point therein.

As used herein, the term "non-polygonal" refers to any shape, two dimensional or three dimensional, having any side being curved or any portion of the shape being curved. Some examples of non-polygonal shapes include (along with their three dimensional counterparts) an oval shape, a substantially oval shape, and arc shape, a substantially arc shape, an arcuate shape, a substantially arcuate shape, a circular shape, a substantially circular shape, a semi-circular shape, a substantially semi-circular shape, a substantially oval shape, an elliptical shape, a substantially elliptical shape, an erratic shape, etc., as non-limiting examples.

As used herein, the term "biocompatible" or "biocompatible material" refers to any material which upon implantation into a mammal's body does not elicit a substantial detrimental response *in vivo,* and/or a material exhibiting essentially no cytotoxicity or immunogenicity while in contact with body fluids, tissues and/or bones. For example, the biocompatible material can be selected from the group consisting of titanium (of any grade), Nitinol^{®}, steel, surgical steel, stainless steel, calcium, copper, zinc, iron, cobalt, magnesium, manganese, vanadium, molybdenum, silicate, strontium, zirconium, tungsten, chromium, tantalum, nickel, aluminum, and ceramics, composites, alloys (such as TiA16V4), compounds, and mixtures thereof.

As used herein, the term "implantable structure" or "implant" or "prosthesis", which can be used interchangeably, can refer to any element that is configured for implantation into a mammalian body, which may or may not contact a bone of that mammal. For example and without limitation, the "implantable structure", and/or "implant", and/or "prosthesis" of the present disclosure can be any portion of (or any component that can be used with) any hip implant/replacement, including an acetabular cup, a bone augment, including a femoral, tibial, acetabular and humeral augment, pelvic implant/replacement, shoulder implant/replacement, including a humeral cup, knee implant/replacement, ankle implant/replacement, elbow implant/replacement, wrist implant/replacement, finger implant/replacement, a revision prosthesis, jaw implants/replacements, dental implants/replacements, spinal implants/replacements, etc. The "implantable structure" may also include a bulkhead element dividing porous implantable structures into distinct sections. The bulkhead element can reduce and/or prevent cement and/or other adhesive applied on one side of the body from reaching the other side of the bulkhead. In this type of embodiment, the porous implantable structure portion can be a reinforcing element, and provide additional mechanical strength to the bulkhead element. The "implantable structure" may also be a cone-like shape having an interior channel running from a bottom to a top side and being configured such as to be enable accommodation of a shank of an endoprosthesis, such as a femur part of a hip endoprosthesis or tibial parts of a knee endoprosthesis. The "implantable structure" may also be an entire, or a portion of, a spinal cage. The "implantable structure" or "implant" or "prosthesis" can be configured to be any suitable size, which can be based on the site for implantation and/or the site for implantation and the size of the patient. A range of sizes of "implantable structure" or "implant" or "prosthesis" can also be created, so that a medical professional can select an appropriate "implantable structure" or "implant" or "prosthesis" for implantation.

Spinal cages are implant devices which can be placed in an intervertebral space between two adjacent vertebrae, in lieu of, or to supplement, a natural vertebral disc. Thereby, spinal cages can prevent a collapse of the intervertebral space. One aim of the spinal cage is to effect fusion of the two adjacent vertebrae by bone material growing in and into the intervertebral space. The spinal cage according to the present disclosure can comprise a porous inner core having a non-polygonal porous structure according to the present disclosure for promoting bone ingrowth. The inner core can be surrounded by an encasement. The encasement can be made of solid material and can serve a double purpose of carrying load forces and protecting the porous structure of the inner core. Further, on a top side and a bottom side of the encasement a plurality of arresting teeth may be provided. The teeth can be pyramidal shaped, and can be configured to substantially arrest the implant at its place. Further, the teeth may be slanted against direction of implantation, thereby facilitating implanting and blocking unwanted dislocation against the direction of implantation.

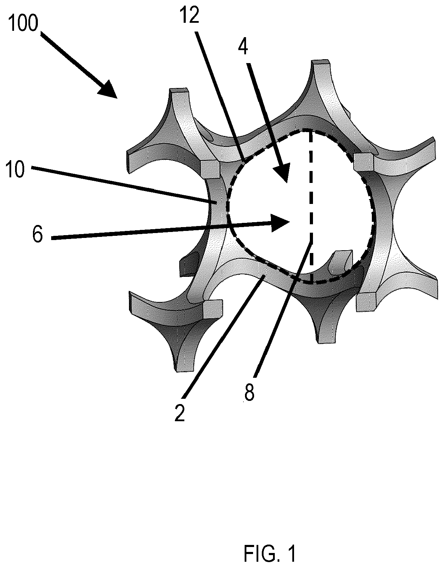

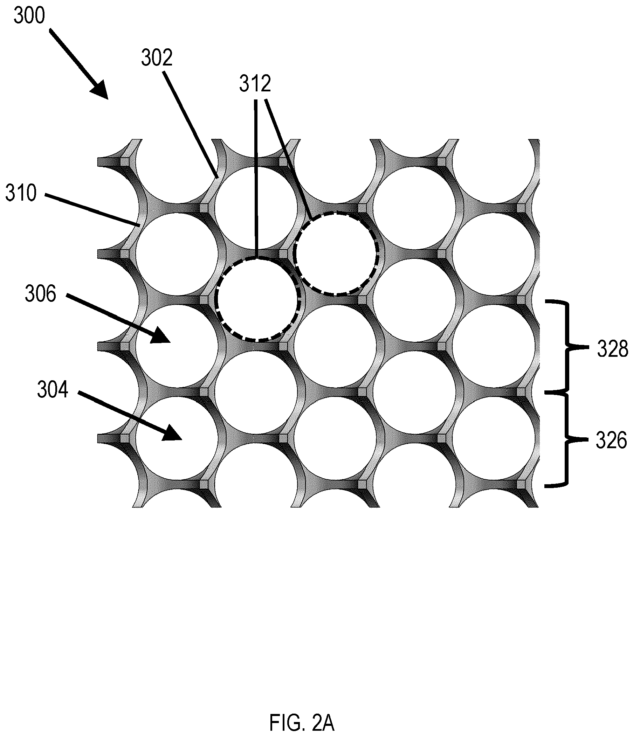

As seen in FIG. 1, a magnified, cross-sectional view of a portion of an embodiment of a porous, implantable structure 100 of the disclosure is shown. The porous, implantable structure 100 includes substantially regularly arranged elementary cells 2, which are substantially arranged within a larger grouping of elementary cells 2, as shown in additional views. These elementary cells 2 comprise interior spaces 4, with the interior spaces forming a plurality of interconnected pores 6. The elementary cells 2 are comprised of basic elements 10, which in FIG. 1, are shown in cross-section of one layer and two partial layers, but as can be seen in FIG. 1, the pore 6 of the illustrated layer is offset from pores in adjacent layers. In further figures the basic elements 10 are shown in several layers, with those layers being offset as compared to adjacent layers. The basic elements 10 are configured to form a non-polygonal shape of each of the plurality of interconnected pores 6. For illustrative purposes, the non-polygonal shape of the interconnected pore 6 of FIG. 1 is shown in a dashed line 12, however as noted herein, other pores of other embodiments can be of other non-polygonal shapes. Additionally, in this embodiment of FIG. 1 the non-polygonal shape of the interconnected pore 6 is substantially circular (as shown in FIG. 2A) but appears ovular due to the perspective angle of FIG. 1.

Such non-polygonal shapes of the interconnected pore 6 of FIG. 1 are more similar in shape to the pores of real, natural bone, including for example trabecular bone material, compared to polygonal shapes. Such similarity in shape results in better biological fixation and bone ingrowth.

The pores 6 (and all pores of the present disclosure) can be dimensioned to any suitable size, such as an average pore width 8 ranging from about 0.1 mm to about 1.5 mm. In other embodiments, an average pore width 8 can range from about 0.4mm to about 1.0mm. In other embodiments, the average pore width 8 can range from about 0.1 mm, or up to about 99% less than about 0.1 mm, to about 1.5 mm, or up to about 100%, about 200%, about 300%, about 400%, or about 500% more than about 1.5 mm. In FIG. 1 the pore width 8 is shown as substantially vertical as pore 6 is substantially circular, thus pore widths in any orientation would be substantially the same. However, in other embodiments of porous, implantable structure 100, the pore width 8 can be any dimension of the pore 6, from a shortest line to a longest line between the basic elements 10.

The basic elements 10 (and all basic elements of the present disclosure) can be dimensioned to have any suitable thickness, such as a thickness be is between about 0.2 mm to about 1.0 mm. In other embodiments, the basic elements 10 can have a thickness between about 0.4 mm and about 0.7 mm. In other embodiments, the average thickness can range from about 0.2 mm, or up to about 99% less than about 0.2 mm, to about 1.0 mm, or up to about 100%, about 200%, about 300%, about 400%, or about 500% more than about 1.0 mm. As seen in the figures, the thickness of the basic elements 10 appears to be substantially uniform, however, the thickness of the basic elements 10 vary minimally or substantially throughout the porous, implantable structure 100.

Any portion, or the entirety of, the porous, implantable structure 100 (and all porous, implantable structures of the present disclosure), including the basic elements 10, can comprise a biocompatible material. Additionally, any portion, or the entirety of, the porous, implantable structure 100, including the basic elements 10, can be coated with any suitable coating (not shown) according to any suitable coating method. The suitable coating can comprise a bone growth promoting material, such as one or more selected from the group consisting of naturally occurring bone material, processed bone material, synthetic bone material, hydroxyapatite comprising material, phosphate comprising materials and compounds thereof, such as beta-tricalcium phosphate, calcium phosphate comprising material and compounds thereof, such as calcium phosphate (CaP), naturally occurring bone morphogenic proteins, recombinant bone morphogenic proteins, synthetic bone morphogenic proteins, growth factors, mineralizing protein comprising material, ossifying protein comprising material, and cytokine comprising material.

This coating can aid in a more rapid and thorough ingrowth of adjacent natural bone material into the porous, implantable structure 100 upon implantation.

Although the coating can be applied in any suitable way, one example of such application is by PVD (Physical Vapor Deposition). The coating can include bone growth related coating alone, or in combination with another PVD material. The PVD material of the PVD coating can be selected from a group comprising niobium, tantalum, zirconium, oxides thereof (niobium, tantalumoxide, and circomium oxide), and combinations thereof.

The coating can be applied in any suitable thickness, such as between about 1 µm and about 10 µm, or about 1 µm and about 100 µm, or about 1 µm and about 1 mm, or about 1 µm and about 5 mm, or about 7 µm.

Any portion, or all of, of any porous, implantable structure disclosed herein can be formed wholly or partially in any suitable way, such as through any suitable additive manufacturing (AM) and/or 3D-printing technology. For example, the basic elements 10 of the porous, implantable structure 100 can be formed according to a suitable electron beam melting (EBM) process and/or a suitable selective laser melting (SLM) process.

EBM is an additive process for manufacturing and may produce solid or porous material. A powder of the desired material is provided in the desired granulometry. By the EBM process the powders of the desired material are deposited in successive layers at desired positions and in desired sequence (as defined in a preceding modelling step for the porous structure) and made to melt such as to form a coherent, solid body, such as any portion of, or the entirety of, any porous, implantable structure 100 disclosed herein.

SLM is a manufacturing technique that uses a laser to fuse metallic or non metallic powders, to form a portion, or the entirety of, the porous, implantable structure 100 (including the basic elements 10). The laser selectively fuses powdered material by scanning cross-sections generated from a 3-Dimensional digital description of the portion, or the entirety of, the porous, implantable structure 100 (including the basic elements 10), for example from a programmed computer file, on the surface of a powder bed. After each cross-section is scanned, the powder bed is lowered by one layer thickness, a new layer of material is applied on top, and the process is repeated until the portion, or the entirety of, the porous, implantable structure 100 manufactured to the desired degree.

As one example of this AM technology, the basic elements 10 of the porous, implantable structure 100 can be additively manufactured from Ti-6Al-4V extra low interstitial (ELI) powder in an electron beam machine (Arcam EBM Q10 plus).

The disclosure further relates to a method for manufacturing a bone implant and comprises that comprises a body (not shown) having a porous, implantable structure 100 and having a size and shape configured for fitting to a bone, wherein the method comprises manufacturing the bone implant by using a depositing technique to: form alternating layers of basic elements 10; and form substantially regularly arranged elementary cells 2 comprising interior spaces 4 that form a plurality of interconnected pores 6, the elementary cells comprising basic elements arranged in layers, wherein the basic elements are configured to form a non-polygonal shape of each of the plurality of interconnected pores 6, wherein each layer is offset with respect to an adjacent layer of the alternating layers. By virtue of this method all embodiments described above may be manufactured.

The above method can further comprise the steps of providing a three-dimensional model of the bone implant, defining a body of the bone implant, defining a bone contacting surface of the bone implant which is configured to complement a corresponding surface of the bone, wherein at least the bone contacting surface is manufactured as a porous, implantable structure 100, with non-polygon shaped pores 6. Thereby a tailor made bone implant manufacture according to specific needs of a patient can be realized. Employing EBM or SLM process enables a precise and effective manufacturing of such implants.

In another embodiment, porous, implantable structure 100 can further comprise a reinforcing structure (not shown), which can be comprises of a solid material. Thereby, robustness in terms of mechanical stress can be further increased, while maintaining the general advantages of the porous, implantable structure 100. The reinforcing structure can be formed to be integral with the porous, implantable structure 100. The reinforcing structure can also comprise a solid body structure provided in addition to the porous, implantable structure 100, wherein the porous, implantable structure 100 is attached to the solid body structure. The solid body structure and the porous, implantable structure 100 can also be formed as a unitary structure.

In another embodiment, an endoprosthetic implant comprising a body made of a solid material and a porous, implantable structure 100 is disclosed. In one embodiment the body is a cup, such as for an acetabulum part of a hip endoprosthesis. By virtue of the cup, a bearing can be formed for engaging a head of a femur part in an articulated manner. In other embodiments, the endoprosthetic implant can be part of a body, wherein the body is a component of an articulated joint. Owing to the combination with the porous, implantable structure 100 a mechanical sound articulation is combined with an anchoring microstructure with improved biocompatibility, in particular in respect to stress loading and load transfer from the cup into the acetabulum.

In some embodiments an entire structure of any implantable structure 100 disclosed herein can be formed through any suitable AM and/or 3D-printing technology. In other embodiments any suitable AM and/or 3D-printing technology can be used to apply one or more elements to an underlying skeletal structure. For example, an underlying skeletal structure can have the basic elements 10 of the implantable structure 100 applied thereto. Thus, a thickness of the basic elements 10 and/or the implantable structure 100, after being applied to an underlying skeletal structure, can be about 1 µm to about 10 mm, about 10 µm to about 5 mm, about 100 µm to about 1 mm, can be about 0.1 mm or less, about 0.5 mm, about 1 mm, about 2 mm, about 3 mm, about 4 mm, about 5 mm or more.

The disclosed porous, implantable structure 100, or any disclosed implantable structure that includes a non-polygonal shape of the plurality of interconnected pores 6, is structurally different from a porous structure having polygon shaped pores, such as a diamond polygonal pore configuration.

One advantage of the porous, implantable structure 100 is apparent if contrasted to another microstructure for bone augments, namely having elementary cells formed in a diamond or diamond-like lattice. Although such a diamond-like structure has good load carrying capability owing to its high Young's modulus, it shows the same high Young's modulus in all cardinal directions. This is a disadvantage in terms of biocompatibility in particular with respect to bone augments, since natural bone possesses different stiffness in different directions. The present disclosure overcomes this disadvantage, with adj acent layers being offset from each other. Thereby the load bearing capability is further improved only in the direction of the extended centerline which is perpendicular to the plane of the layers.

As discussed above, FIG. 1 is a magnified view of a portion of a structure, FIGS. 2A-2C are also magnified views and are of a greater portion of the porous, implantable structure shown in FIG. 1. FIG. 2A is a magnified, cross-sectional view of an embodiment of a porous, implantable structure 300 of the disclosure, which includes repeated elements of what is shown in FIG. 1. The porous, implantable structure 300 includes substantially regularly arranged elementary cells 302, which are substantially arranged within a larger grouping of elementary cells 302. These elementary cells 302 comprise interior spaces 304, with the interior spaces forming a plurality of interconnected pores 306. The elementary cells 302 are comprised of basic elements 310, which in FIG. 2A, are shown in cross-section of four layers in the X-Y plane and four partial layers in the X-Y plane. Two adjacent layers are indicated as lower layer 326 and upper layer 328, the pores 306 of which are offset from each other, as seen in FIG. 2A, in the X-Y plane.

In FIGs. 2A-2C the basic elements 310 are shown in several layers, with those layers being offset as compared to adjacent layers. The basic elements 310 are configured to form a non-polygonal shape of each of the plurality of interconnected pores 306. For illustrative purposes, the non-polygonal shape of the interconnected pore 306 of FIG. 2A is shown in a dashed line 312, however as noted herein, other pores of other embodiments can be of other non-polygonal shapes. In this embodiment of FIG. 2A the non-polygonal shape of the interconnected pore 306 is substantially circular.

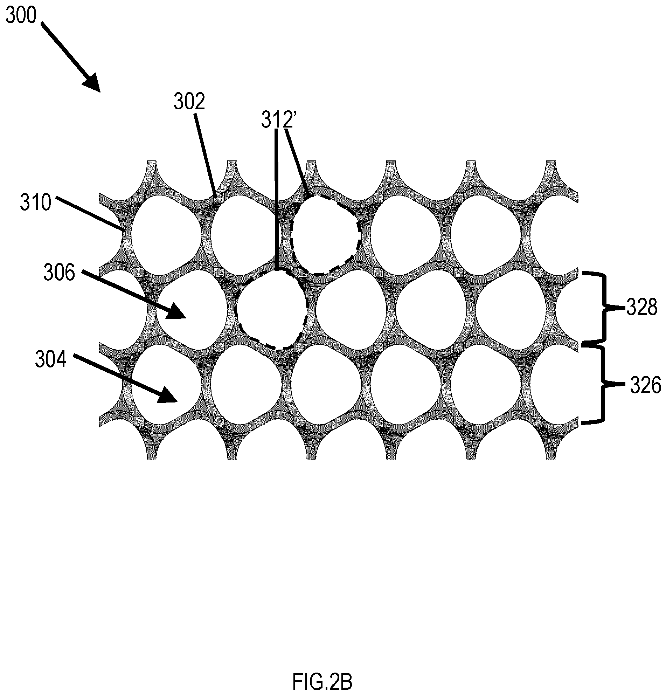

Another view of the embodiment of the porous, implantable structure 300 is shown in FIG. 2B, which is a view along the Y-Z plane of the structure shown in FIG. 1. In this embodiment, the non-polygonal shape of the interconnected pore 306', shown in a dashed line 312, is substantially ovular, however as noted herein, other pores of other embodiments can be of other non-polygonal shapes.

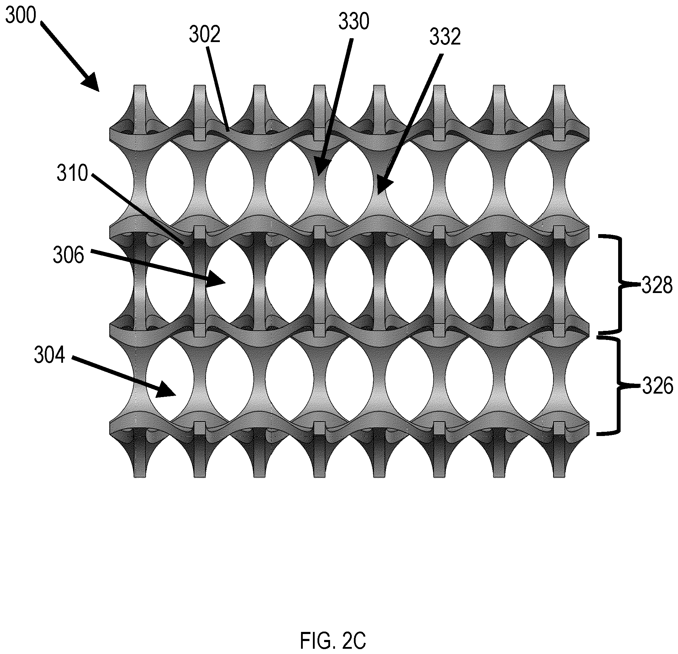

Another embodiment of the porous, implantable structure 300 is shown in FIG. 2C, which is a view along the X-Z plane of the structure shown in FIG. 1, with FIG. 2C illustrating a depth of two layer in the Y direction as well as three layers in the X-Z plane (including layers 326 and 328) and two partial layers in the X-Z plane. In the Y direction a distal layer 330 and a proximal layer 332 are seen. Thus, the porous, implantable structure 300 (or any porous, implantable structure of the present disclosure) can include layers that are offset with respect to adjacent layers in the X-Z plane (such as layers 326 and 328) and/or include layers that are offset with respect to adjacent layers in the Z direction (such as layers 330 and 332).

The described embodiments and examples of the present disclosure are intended to be illustrative rather than restrictive, and are not intended to represent every embodiment or example of the present disclosure. While the fundamental novel features of the disclosure as applied to various specific embodiments thereof have been shown, described and pointed out, it will also be understood that various omissions, substitutions and changes in the form and details of the devices illustrated and in their operation, may be made by those skilled in the art without departing from the spirit of the disclosure. For example, it is expressly intended that all combinations of those elements and/or method steps which perform substantially the same function in substantially the same way to achieve the same results are within the scope of the disclosure. Moreover, it should be recognized that structures and/or elements and/or method steps shown and/or described in connection with any disclosed form or embodiment of the disclosure may be incorporated in any other disclosed or described or suggested form or embodiment as a general matter of design choice. Further, various modifications and variations can be made without departing from the spirit or scope of the disclosure as set forth in the following claims both literally and in equivalents recognized in law.

Detailed embodiments of the present prostheses and implant devices and methods are disclosed herein; however, it is to be understood that the disclosed embodiments are merely illustrative and that the prostheses and implant devices and methods may be embodied in various forms. In addition, each of the examples given in connection with the various embodiments of the systems and methods are intended to be illustrative, and not restrictive.

Throughout the specification, where compositions are described as including components or materials, it is contemplated that the compositions can also consist essentially of, or consist of, any combination of the recited components or materials, unless described otherwise. Likewise, where methods are described as including particular steps, it is contemplated that the methods can also consist essentially of, or consist of, any combination of the recited steps, unless described otherwise. The invention illustratively disclosed herein suitably may be practiced in the absence of any element or step which is not specifically disclosed herein.

The practice of a method disclosed herein, and individual steps thereof, can be performed manually and/or with the aid of or automation provided by electronic equipment. Although processes have been described with reference to particular embodiments, a person of ordinary skill in the art will readily appreciate that other ways of performing the acts associated with the methods may be used. For example, the order of various steps may be changed without departing from the scope or spirit of the method, unless described otherwise. In addition, some of the individual steps can be combined, omitted, or further subdivided into additional steps.

## Claims

1. A porous, implantable structure, comprising:
substantially regularly arranged elementary cells, wherein the elementary cells comprise interior spaces that form a plurality of interconnected pores, the elementary cells comprise basic elements arranged in layers, wherein the basic elements are configured to form a non-polygonal shape of each of the plurality of interconnected pores, wherein each layer is offset with respect to an adjacent layer.

2. The porous, implantable structure of claim 1, wherein the basic elements comprise a biocompatible material.

3. The porous, implantable structure of claim 1 or claim 2, wherein at least a portion of the structure is coated with a bone growth promoting material.

4. The porous, implantable structure of any one of the preceding claims, wherein the basic elements are made in place through deposition and solidification.

5. The porous, implantable structure of any one of the preceding claims, wherein the interconnected pores comprise a pore width, wherein the pore width is between about 0.1 mm to about 1.5 mm, or between about 0.4 mm to about 1.0 mm, or between about 0.2 mm to about 1.0 mm, or between about 0.4 mm to about 0.7 mm.

6. An endoprosthetic implant comprising:
a body made of a solid material and a bone contacting portion made of a porous, implantable structure, the porous, implantable structure according to any one of the preceding claims.

7. The endoprosthetic implant of claim 6, wherein the body is a component of an articulated joint.

8. The endoprosthetic implant of claim 6 or claim 7, wherein the body is a bulkhead element dividing the porous, implantable structure into distinct sections.

9. The endoprosthetic implant of any one of claims 6 to 8, wherein the body is a reinforcing element.

10. The endoprosthetic implant of claim 6 or claim 7, wherein the body is a component of a cup.

11. The endoprosthetic implant of claim 8, wherein the bulkhead element is configured to block cement from flowing across.

12. A method for manufacturing a bone implant that comprises a body having a porous, implantable structure and having a size and shape configured for fitting to a bone, wherein the method comprises manufacturing the bone implant by using a depositing technique to:
form substantially regularly arranged elementary cells comprising interior spaces that form a plurality of interconnected pores, the elementary cells comprising basic elements arranged in alternating layers, wherein the basic elements are configured to form a non-polygonal shape of each of the plurality of interconnected pores, wherein each layer of the alternating layers is offset with respect to an adjacent layer of the alternating layers.

13. The method of claim 12, further comprising depositing a coating on at least a portion of the porous, implantable structure by a Physical Vapor Deposition (PVD) process.

14. The method of claim 13, wherein the coating comprises tantalum.

15. The method of any one of claims 12 to 14, further comprising depositing a CaP on at least a portion of the porous, implantable structure.
